(19) Europäisches Patentamt / European Patent Office / Office européen des brevets

(11) **EP 4 682 896 A1**

(12) **EUROPEAN PATENT APPLICATION**
published in accordance with Art. 153(4) EPC

(43) Date of publication:
**21.01.2026 Bulletin 2026/04**

(21) Application number: **24815875.0**

(22) Date of filing: **30.05.2024**

(51) International Patent Classification (IPC):
$G16H\ 10/60^{(2018.01)}$    $G16H\ 50/20^{(2018.01)}$
$G16H\ 40/60^{(2018.01)}$    $A61B\ 5/021^{(2006.01)}$
$A61B\ 5/00^{(2006.01)}$    $G06N\ 3/0464^{(2023.01)}$
$G06N\ 3/044^{(2023.01)}$    $G06N\ 3/0475^{(2023.01)}$

(52) Cooperative Patent Classification (CPC):
A61B 5/00; A61B 5/021; G06N 3/044;
G06N 3/0464; G06N 3/0475; G16H 10/60;
G16H 40/60; G16H 50/20

(86) International application number:
**PCT/KR2024/007392**

(87) International publication number:
**WO 2024/248501 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.05.2023 KR 20230069381**

(71) Applicant: **Medical AI Co., Ltd.**
**Seoul 06180 (KR)**

(72) Inventors:
• **KWON, Joonmyoung**
  **Seoul 06180 (KR)**
• **LEE, Byeongtak**
  **Seoul 06180 (KR)**
• **JO, Yongyeon**
  **Seoul 06180 (KR)**

(74) Representative: **Patentanwaltskanzlei
Matschnig & Forsthuber OG
Biberstraße 22
Postfach 36
1010 Wien (AT)**

(54) **METHOD, PROGRAM, AND DEVICE FOR ACQUIRING USER-CUSTOMIZED NEURAL NETWORK MODEL FOR IDENTIFYING BIOMETRIC INFORMATION**

(57) According to one embodiment of the present disclosure, there is disclosed a method of acquiring a user-customized neural network model that predicts bio-information of a user, the method being performed by a computing device including at least one processor, the method including: acquiring a neural network model trained to predict second bio-information of a different type from that of first bio-information based on the first bio-information; and, when new data for the user is acquired, updating the neural network model based on the acquired new data so that the neural network model is personalized for the user; wherein the new data includes at least one of first bio-information for the user and second bio-information for the user.

Fig. 1

**Description**

**Technical Field**

The present disclosure relates to deep learning technology in the medical field, and more particularly, to a method, program, and device for acquiring a user-customized neural network model that identifies bio-information.

**Background Art**

With the recent development of information and communication technology, deep learning technology is being utilized in various fields. In particular, deep learning technology is also being used in a variety of ways in the medical field, which has traditionally relied on specialized and limited personnel such as doctors, researchers, etc. to diagnose diseases of patients. For example, users can have their health status analyzed or their diseases diagnosed in real time without the need for a visit to a specialized medical institution by inputting their bio-information to a pre-trained deep learning model. Furthermore, interest in real-time patient monitoring using deep learning technology has grown as one of the schemes for reducing the labor costs incurred for patient care.

Meanwhile, a deep learning model used in the medical field is pre-trained based on pre-set training data and provided to individual users. Accordingly, in real-world applications, a problem arises in that the deep learning model may sometimes produce different results for individual users or subjects (e.g., patients). The reason for this is that even bio-information for the same type or status may be acquired in different forms or as different values for individual users. This is also caused by the biological characteristics of individual users. Therefore, there is a demand for a scheme for personalizing even the same deep learning model for each user by reflecting the environment and characteristics of the user therein.

**Disclosure**

**Technical Problem**

The present disclosure has been conceived in response to the above-described background art, and an object of the present invention is to provide a method, program, and device for acquiring a user-customized neural network model that identifies bio-information.

However, the objects to be achieved in the present disclosure are not limited to the object mentioned above, and other objects not mentioned may be clearly understood based on the following description.

**Technical Solution**

According to one embodiment of the present disclosure for achieving the above-described object, there is disclosed a method of acquiring a user-customized neural network model that predicts bio-information of a user, the method being performed by a computing device including at least one processor, the method including: acquiring a neural network model trained to predict second bio-information of a different type from that of first bio-information based on the first bio-information; and, when new data for the user is acquired, updating the neural network model based on the acquired new data so that the neural network model is personalized for the user; wherein the new data includes at least one of first bio-information for the user and second bio-information for the user.

Alternatively, the updating may include: continuously acquiring first bio-information for the user; and updating the neural network model by performing first learning on the neural network model based on the acquired first bio-information.

Alternatively, the updating may include: acquiring second bio-information for the user while continuously acquiring first bio-information for the user; and updating the neural network model by performing second learning on the neural network model based on the acquired second bio-information and first bio-information corresponding to the acquired second bio-information out of the continuously acquired first bio-information.

Alternatively, updating the neural network model by performing second learning on the neural network model may include performing the second learning on the neural network model based on the acquired second bio-information and first bio-information corresponding to the acquired second bio-information each time the second bio-information is acquired;

Alternatively, updating the neural network model by performing second learning on the neural network model may include: when the second bio-information is acquired, updating a dataset accumulated and acquired at a previous time by adding the acquired second bio-information and first bio-information corresponding to the acquired second bio-information to the dataset; and performing second learning on the neural network model based on the updated dataset; wherein the dataset includes at least one piece of second bio-information accumulated and acquired at the previous time and at least one first piece bio-information corresponding to the at least one piece of second bio-information, which are matched with each other.

Alternatively, the first bio-information corresponding to the second bio-information may be acquired within a preset time range from the time when the second bio-information was acquired.

Alternatively, the method may include acquiring predicted second bio-information by inputting first bio-information corresponding to the acquired second bio-information to the neural network model, and determining whether to stop a process of updating the neural network model by comparing the acquired second bio-information with the predicted second bio-information.

Alternatively, the method may include identifying biological information of the user, clustering the user and a plurality of other users based on the identified biological information, and assigning a dataset, including the new data, to each cluster.

Alternatively, the first learning may be a self-supervised learning method based on the acquired first bio-information.

Alternatively, the second learning may be a supervised learning method based on the acquired second bio-information and first bio-information corresponding to the acquired second bio-information.

Alternatively, the first bio-information may include bio-information acquired from the user based on a non-invasive measurement method, and the second bio-information may include bio-information acquired from the user based on an invasive measurement method or bio-information acquired through direct measurement by a medical professional.

According to one embodiment of the present disclosure for achieving the above-described object, there is disclosed a method of diagnosing a disease of a user based on a user-customized neural network model, the method being performed by a computing device including at least one processor, the method including: acquiring first bio-information; and identifying second bio-information of a different type from that of the first bio-information by inputting the acquired first bio-information to a pre-trained neural network model; wherein the pre-trained neural network model is updated based on new data each time the new data for the user is acquired; and wherein the new data includes at least one of first bio-information for the user and second bio-information for the user.

According to one embodiment of the present disclosure for achieving the above-described object, there is disclosed a computer program stored in a computer-readable storage medium, the computer program causing operations for acquiring a user-customized neural network model that predicts bio-information of a user to be performed when executed by at least one processor, wherein the operations include operations of: acquiring a neural network model trained to predict second bio-information of a different type from that of first bio-information based on the first bio-information; and, when new data for the user is acquired, updating the neural network model based on the acquired new data so that the neural network model is personalized for the user; wherein the new data includes at least one of first bio-information for the user and second bio-information for the user.

According to one embodiment of the present disclosure for achieving the above-described object, there is disclosed a computing device for acquiring a user-customized neural network model that predicts bio-information of a user, the computing device including: memory including program codes; a network unit; a sensing unit configured to detect first bio-information of a user; and a processor configured to acquire a neural network model trained to predict second bio-information of a different type from that of first bio-information based on the first bio-information, and to, when new data for the user is acquired, update the neural network model based on the acquired new data so that the neural network model is personalized for the user; wherein the new data includes at least one of first bio-information for the user and second bio-information for the user.

**Advantageous Effects**

According to the method of acquiring a user-customized neural network model that identifies bio-information according to one embodiment of the present disclosure, it may be possible to obtain a neural network model that identifies more accurate bio-information for a user by reflecting the characteristics of the user therein. Furthermore, it may be possible to personalize a neural network model stored in a computing device to suit a user simply by acquiring bio-information for the user.

**Description** of Drawings

FIG. 1 is an exemplary diagram of a device for acquiring a user-customized neural network model that predicts bio-information according to one embodiment of the present disclosure;
FIG. 2 is a block diagram of a computing device according to one embodiment of the present disclosure;
FIG. 3 is a flowchart schematically showing a method of acquiring a user-customized neural network model that predicts bio-information according to one embodiment of the present disclosure;

FIG. 4 is an exemplary diagram showing updating a neural network model based on first and second bio-information according to one embodiment of the present disclosure;

FIG. 5 is a flowchart showing updating a neural network model based on first and second bio-information according to one embodiment of the present disclosure;

FIG. 6 is an exemplary diagram showing updating the neural network model based on first bio-information and a plurality of pieces of second bio-information included in a dataset according to one embodiment of the present disclosure; and

FIG. 7 is a block diagram of a computing device according to another embodiment of the present disclosure.

## Mode for Invention

Embodiments of the present disclosure will be described in detail below with reference to the accompanying drawings so that those having ordinary skill in the art of the present disclosure (hereinafter, those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Furthermore, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquiring" used herein may be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Furthermore, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The "data" used herein may include an "image," a signal, and the like. The term "image" used herein may refer to multidimensional data composed of discrete image elements. In other words, the "image" may be understood as a term referring to a digital representation of an object that is visible to the human eye. For example, the term "image" may refer to multidimensional data composed of elements corresponding to pixels in a two-dimensional image. The term "image" may refer to multidimensional data composed of elements corresponding to voxels in a three-dimensional image.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

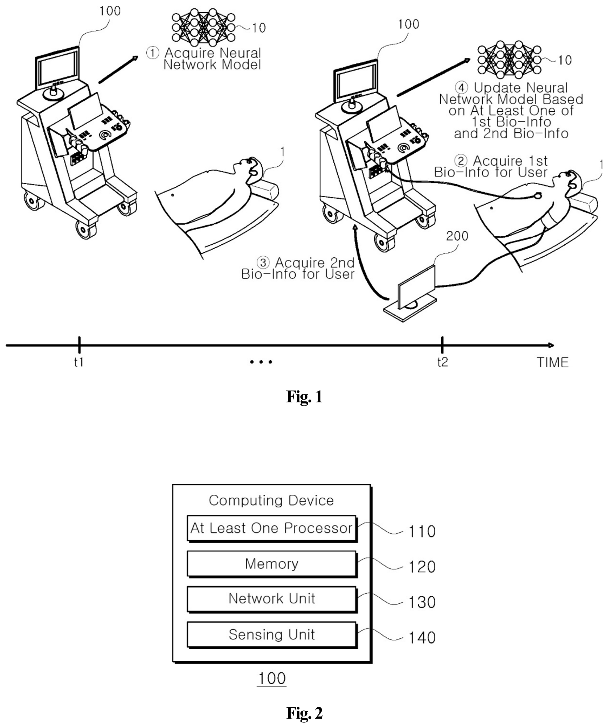

FIG. 1 is an exemplary diagram of a device for acquiring a user-customized neural network model 10 that predicts bio-information according to one embodiment of the present disclosure.

A computing device 100 according to one embodiment of the present disclosure may be a hardware device or a part of a hardware device that performs the comprehensive processing and computation of data, or may be a software-based computing environment connected over a communication network. For example, the computing device 100 may be a server that is a main agent for performing an intensive data processing function and sharing resources, or may be a client that shares resources through interaction with a server. Alternatively, the computing device 100 may be a cloud system in which multiple servers and clients comprehensively process data while interacting with each other. Since the above description is only one example related to the type of computing device 100, the type of computing device 100 may be configured in various manners within a range understandable to those skilled in the art based on the content of the present disclosure.

Referring to FIG. 1, the computing device 100 according to one embodiment of the present disclosure may be implemented as a biosignal measurement device that measures bio-information of a user 1 (e.g., a patient, or the like). However, the computing device 100 is not limited thereto, and may be implemented as various electronic devices, such as a desktop, a laptop, a smartphone, a server device, a smartwatch, a smart band, a smart ring, and the like.

Meanwhile, the pre-trained neural network model 10 may be stored in the computing device 100. The neural network model 10 may be a model pre-trained to predict the status, symptom, disease, bio-information, and/or the like of the user 1. In particular, the neural network model 10 may be pre-trained in accordance with the type, purpose of use, and/or the like of the computing device 100.

According to one embodiment of the present disclosure, the neural network model 10 may be pre-trained to predict another piece of bio-information of the user 1 based on the bio-information acquired from the user 1 by the computing device 100. In this case, the bio-information may include biosignals (e.g., an electrocardiogram (ECG) signal, an electroencephalogram (EEG) signal, a photoplethysmogram (PPG) signal, a disease type (e.g., heart failure, or the like) and disease probability, and measured values (e.g., body temperature, blood pressure (particularly, arterial blood pressure (ABP), central venous pressure (CVP), left ventricular ejection fraction (EF), and the like)) acquired for the user 1.

In the following description, for ease of description of the present disclosure, the bio-information directly acquired by the computing device 100 and input to the neural network model 10 will be referred to as first bio-information. Furthermore, the bio-information predicted by the neural network model 10 by using the first bio-information as input data will be referred to as second bio-information.

The first bio-information and the second bio-information may be different types of bio-information. In particular, according to one embodiment of the present disclosure, the first bio-information may include the bio-information acquired from the user 1 by using a non-invasive measurement method. In particular, the first bio-information may be the bio-information acquired from the user 1 in real time. As an example, the first bio-information may include an electrocardiogram signal, an electroencephalogram signal, a photoplethysmogram signal, and the like. In contrast, the second bio-information may include the bio-information acquired from the user 1 by using an invasive measurement method or the bio-information acquired through direct measurement by a medical professional. As an example, the second bio-information may include body temperature, blood pressure (e.g., arterial blood pressure, central venous blood pressure, and/or the like), left ventricular ejection fraction, and/or the like.

That is, the neural network model 10 may be trained to identify the second bio-information acquired based on a non-invasive method which requires the intervention of a medical professional or by which it is generally difficult to measure information by using the first bio-information acquired based on a non-invasive method by which it is easy to measure information from the user 1. This enables the monitoring of secondary bio-information of the user 1 and the real-time examination of the status of the user 1 simply by using only the computing device 100 without the intervention of a professional such as a doctor or a nurse. In particular, there is an advantage in which the amount of nursing staff required for patient care is reduced in that it is possible to monitor second bio-information of patients requiring continuous care in real time.

Meanwhile, the neural network model may be implemented as a multi-layer perceptron (MLP), a convolutional neural network (CNN), a recurrent neural network (RNN), a generative adversarial network, or the like. In particular, the neural network model may include a plurality of residual blocks configured to extract latent features of the input first bio-information, and a classifier configured to classify second bio-information or a specific disease based on the extracted latent features.

Meanwhile, the computing device 100 may be manufactured with a neural network model 10 stored therein. Alternatively, the computing device 100 may acquire the neural network model 10 from an external computing device (e.g., a server device, or the like) that operates in conjunction with the computing device 100. In this case, the computing device 100 may update the neural network model 10 stored therein to suit the user 1. More specifically, the neural network model 10 acquired by the computing device 100 may be a model pre-trained based on a preset training dataset. In this case, the computing device 100 may update the neural network model to suit the user 1 by retraining it based on the new data acquired from the user 1.

In this case, updating the neural network model 10 may involve calibrating the neural network model 10 to suit the user based on the bio-information acquired by the computing device 100. As an example, the computing device 100 may perform the learning process of fine-tuning the neural network model 10 based on the acquired bio-information.

For example, referring to FIG. 1, when the neural network model 10 is a model trained to predict second bio-information based on first bio-information, the computing device 100 may update the neural network model 10 by retraining it based on the first bio-information acquired from the user 1. More specifically, in the case where the first bio-information is an electrocardiogram signal and the second bio-information is blood pressure, when an electrocardiogram signal of the user 1 is acquired, the computing device 100 may input the acquired electrocardiogram signal to the neural network model 10 to predict the blood pressure of the user, and may train the neural network model 10 based on the acquired electrocardiogram signal to update the neural network model 10 to suit the user 1. By repeating this process, the computing device 100 may acquire the personalized neural network model 10 (i.e., the user-customized neural network model 10) that more accurately predicts second bio-information of the user 1 based on the first bio-information of the user 1.

Meanwhile, referring to FIG. 1, the computing device 100 may also acquire second bio-information. The acquired second bio-information is the information acquired by directly measuring the user 1, and is distinct from the second bio-information predicted by inputting first bio-information to the neural network model 1. The second bio-information may be acquired directly by the computing device 100, or may be acquired from an external computing device 200 (e.g., an external biosignal measurement device) and transmitted to the computing device 100. In this case, the computing device 100 may update the neural network model 10 based on the first bio-information acquired by directly measuring the user 1 together with the acquired second bio-information. In particular, the acquired second bio-information may serve as labeled training data, so that the computing device 100 can update the neural network model 10 to more accurately predict second bio-information for the user 1 by using the first and second bio-information.

That is, according to one embodiment of the present disclosure, the learning method in the case where only the first bio-information is acquired and the learning method in the case where the second bio-information is acquired together with the first bio-information may be different.

In the following description, one embodiment of the present disclosure will be described in detail with reference to FIGS. 2 to 6.

FIG. 2 is a block diagram of a computing device 100 according to one embodiment of the present disclosure.

Referring to FIG. 2, the computing device 100 according to one embodiment of the present disclosure may include at least one processor (hereinafter, the "processor") 110, memory 120, a network unit 130, and a sensing unit 140. However, FIG. 1 is merely an example, and the computing device 100 may further include other components for implementing a computing environment. Furthermore, only some of the disclosed components may be included in the computing device 100.

The processor 110 according to one embodiment of the present disclosure may be understood as a constituent unit including hardware and/or software for performing computing operations. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), and a field programmable gate array (FPGA). Since the types of processor 110 described above are only examples, the type of processor 110 may be configured in various manners within a range understandable to those skilled in the art based on the content of the present disclosure.

The processor 110 is electrically connected to other components (i.e., the memory 120, network unit 130, and sensing unit 140) of the computing device 100, and controls the overall operation of the computing device 100.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage the data required for the processor 110 to perform operations, combinations of the data, and program codes executable by the processor 110. For example, the memory 120 may store a neural network model 10 trained to predict second bio-information based on first bio-information. Furthermore, the memory 120 may store program codes configured to operate the neural network model 10 to perform training based on acquired first and second bio-information, program codes configured to operate the neural network model 10 to receive first and second bio-information and perform inference in accordance with the purpose of use of the computing device 100, and processed data that is generated as the program codes are executed.

The network unit 130 according to one embodiment of the present disclosure may be understood as a constituent unit that transmits and receives data via any type of known wired/wireless communication system. For example, the network unit 130 may perform data transmission and reception by using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, a Bluetooth network, or the like. Since the above-described communication systems are only examples, the wired/wireless communication system for the data transmission and reception of the network unit 130 may be applied in various manners other than the above-described examples.

The network unit 130 may receive the data required for the processor 110 to perform operations through wired/wireless communication with any system, any client, or the like. Furthermore, the network unit 130 may transmit the data generated through the operations of the processor 110 through wired/wireless communication with any system, any client, or the like. For example, the network unit 130 may receive medical data through communication with a database within a hospital environment, a cloud server for performing tasks such as the standardization of medical data, a computing device, or the like. The network unit 130 may transmit the output data of the neural network model 10, the intermediate data derived from the computational process of the processor 110, and processed data through communication with the above-described database, server, or computing device, or the like. As an example, the processor 110 may acquire the neural network model 10 trained to predict second bio-information based on first bio-information from an external computing device (e.g., an external server device) via the network unit 130. Furthermore, the processor 110 may acquire second bio-information of the user 1 from an external computing device (e.g., another biosignal measurement device) via the network unit 130.

The sensing unit 140 may acquire first bio-information for the user 1. In particular, the sensing unit 140 may acquire first bio-information for the user 1 by using a non-invasive method. That is, according to one embodiment of the present disclosure, the first bio-information may be bio-information measured according to a non-invasive method. For example, the sensing unit 140 may include a plurality of electrodes. In this case, the processor 110 may acquire an electrocardiogram signal of the user 1 as the first bio-information via at least one electrode. Furthermore, the sensing unit 140 may include an image sensor or an optical sensor. In this case, the processor 110 may acquire a photoplethysmogram signal of the user 1 as the first bio-information via the image sensor (or the optical sensor). Furthermore, the sensing unit 140 may further include a sensing module for second bio-information that can be acquired by a medical professional directly measuring the second bio-information. For example, the sensing unit 140 may further include a blood pressure gauge, a thermometer, and/or the like.

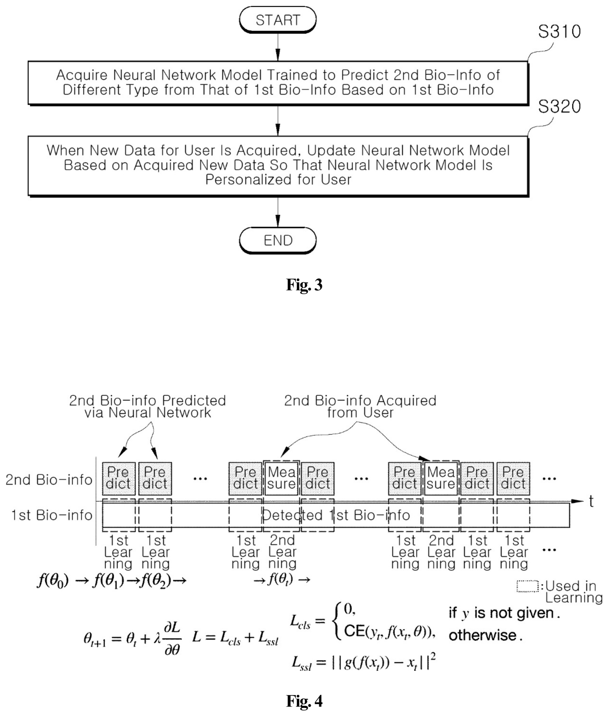

FIG. 3 is a flowchart schematically showing a method of acquiring a user-customized neural network model 10 that predicts bio-information according to one embodiment of the present disclosure.

Referring to FIG. 3, first, the processor 110 acquires a neural network model trained to predict second bio-information of a different type from that of the first bio-information based on the first bio-information in step S310.

More specifically, the processor 110 may acquire the neural network model 10 stored in the memory 120. In this case, the neural network model 10 may be stored in the memory 120 at the time when the computing device 100 is manufactured.

Furthermore, a plurality of neural network models 10 may be stored in the memory 120 of the computing device 100. More specifically, a plurality of neural network models 10 corresponding to a plurality of users 1 (e.g., patients, or the like) may be pre-stored in the memory 120. The processor 110 may select and acquire a neural network model 10 corresponding to the user 1 from among the plurality of neural network models 10 based on the identification information (e.g., the name, resident registration number, or like) (or the bio-information) of the user 1. For example, when the computing device 100 is a biosignal measurement device installed at a specialized medical institution, the computing device 100 may store a plurality of neural network models 10 corresponding to a plurality of patients that have visited the specialized medical institution. Each of the neural network models 10 may be repeatedly updated based on the first and second bio-information acquired for each patient and stored in the memory 120 according to an embodiment of the present disclosure to be described below.

Meanwhile, the neural network model 10 may be trained to predict a plurality of pieces of second bio-information based on the first bio-information. More specifically, the first bio-information may be an electrocardiogram signal, and the plurality of pieces of second bio-information may be blood pressure (second-first bio-information) and left ventricular ejection fraction (second-second bio-information). In this case, the neural network model 10 may extract specific information corresponding to blood pressure (second-first bio-information) from the first bio-information to predict blood pressure (second-first bio-information). Furthermore, the neural network model 10 may extract specific information corresponding to left ventricular ejection fraction (second-second bio-information) to predict left ventricular ejection fraction (second-second bio-information). In this case, the neural network model 10 may include a plurality of residual blocks configured to extract latent features of blood pressure (second-first bio-information) and left ventricular ejection fraction (second-second bio-information), and a plurality of classifiers configured to classify blood pressure (second-first bio-information) and left ventricular ejection fraction (second-second bio-information) based on the extracted latent features.

Furthermore, the memory 120 of the computing device 100 may store a plurality of neural network models 10 corresponding to a plurality of pieces of first bio-information. More specifically, the memory 120 may store a plurality of neural network models 10 each corresponding to each piece of first bio-information and each piece of second bio-information predicted in relation to the first bio-information. For example, a neural network model (a first neural network model) configured to predict the left ventricular ejection fraction of the user 1 as second bio-information based on an electrocardiogram signal as first bio-information, and a neural network model (a second neural network model) configured to predict the blood pressure of the user 1 as second bio-information based on a photoplethysmogram signal as first bio-information may be stored. In this case, the processor 110 may select and acquire a specific neural network model (i.e., a neural network model corresponding to the first bio-information) from among a plurality of neural network models according to the type of first bio-information that is measured (or is set to be measured) by the computing device 100 in real time.

Meanwhile, the processor 110 may also acquire the neural network model 10 from an external computing device (e.g., a server device, or the like) that operates in conjunction with the computing device 100 via the network unit 130. In this case, the external computing device may be a server device that monitors the health status of the user 1 of the computing device 100 while operating in conjunction with the computing device 100. Alternatively, the external computing device may be a biosignal measurement device that measures second bio-information of the user 1.

In addition, when new data for the user is acquired, the processor 110 may update the neural network model based on the acquired new data so that the neural network model can be personalized for the user in step S320.

In this case, the new data may include at least one of the first bio-information of the user 1 and the second bio-information of the user 1, which are acquired by the processor 110 through the sensing unit 140. The first and second bio-information included in the new data are distinct from the first and second bio-information included in the training dataset previously used to train the neural network model 10 in that they were acquired through direct measurement for the user.

According to one embodiment of the present disclosure, the processor 110 may continuously acquire first bio-information for the user 1. The processor 110 may then update the neural network model by performing first learning on the neural network model 10 based on the acquired first bio-information. The process of updating a neural network model may be performed in parallel with the process of predicting second bio-information based on the first bio-information continuously acquired by the processor 110. That is, when the first bio-information is continuously acquired, the processor 110 may predict second bio-information by inputting the continuously acquired first bio-information to the neural network model 10, and may provide the predicted second bio-information to the user 1. Furthermore, the processor 110 may update the neural network model 1 by using the first bio-information continuously acquired in parallel and the second bio-information (, or a loss function, a backpropagation algorithm, or the like) predicted via the first bio-information.

More specifically, the processor 110 may continuously acquire first bio-information for the user 1 via the sensing unit 140. For example, the processor 110 may set a plurality of sample points (e.g., 125 points or 250 points) over a preset period of time (e.g., 1 second) and continuously acquire first bio-information for the user 1 across the plurality of sample points. When the first bio-information is an electrocardiogram signal, the processor 110 may acquire the electrocardiogram signal by measuring it at a sampling rate of 500 Hz for 10 seconds via the sensing unit 140. In this case, the processor 110 may process the electrocardiogram signal by removing the one-second period where the electrocardiogram signal begins and the last one-second period where the electrocardiogram signal ends in order to remove noise or artifacts contained in the electrocardiogram signal.

Furthermore, the processor 110 may continuously acquire first bio-information of the user 1 via the sensing unit 140, and may acquire a plurality of pieces of first bio-information by dividing the continuous first bio-information based on the set sample points.

Furthermore, the processor 110 may periodically acquire first bio-information from an external computing device. For example, when the computing device 100 is implemented as a server device, the processor 110 may continuously receive first bio-information acquired from an external biosignal measurement device that comes into contact with the body of the user 1 and measures and acquires the first bio-information. In this case, the processor 110 may receive the first bio-information from the external biosignal measurement device via the network unit 130.

When first bio-information is acquired, the processor 110 may update the neural network model 10 by performing first learning on the neural network model 10 based on the acquired first bio-information of the user 1. That is, the processor 110 may calibrate the pre-trained neural network model 10 to suit the user 1 based on the first bio-information.

For example, when the processor 110 periodically acquires first bio-information, the processor 110 may perform first learning on the neural network model 10 by using the acquired first bio-information each time the first bio-information is acquired. That is, the first learning may be performed on the neural network model in accordance with the cycle at which the first bio-information is acquired.

According to one embodiment of the present disclosure, the first learning may be self-supervised learning based on the acquired first bio-information. More specifically, the processor 110 may extract latent features from the acquired first bio-information, may assign a virtual label to the first bio-information based on the extracted latent features, and may then train the neural network model 10 based on the first bio-information assigned with the virtual label. For example, the processor 110 may acquire converted first bio-information by preprocessing the first bio-information. The processor 110 may acquire a plurality of pieces of second bio-information predicted based on the latent features extracted for each of the first bio-information and the converted first bio-information by inputting the first bio-information and the converted first bio-information to the pre-trained neural network model 10. The processor may then train the neural network model 10 to minimize the loss between the acquired the pieces of second bio-information based on a loss function. In this case, the loss function may utilize a contrastive loss or an info NCE loss function. The processor 110 may adjust the parameters of the pre-trained neural network model 10 by using a backpropagation algorithm.

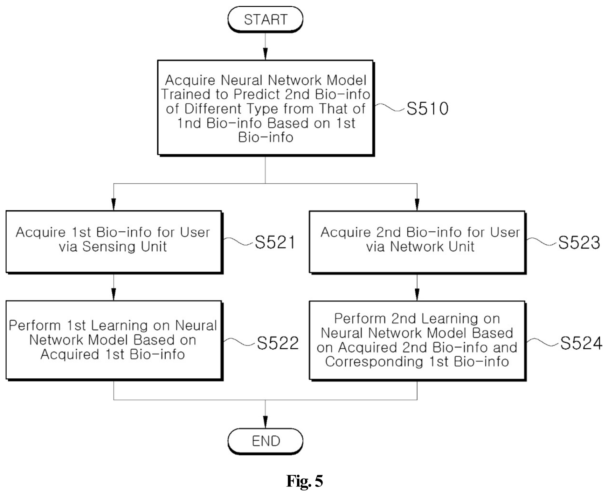

FIG. 4 is an exemplary diagram showing updating a neural network model 10 based on first and second bio-information according to one embodiment of the present disclosure. FIG. 5 is a flowchart showing updating a neural network model based on first and second bio-information according to one embodiment of the present disclosure.

Meanwhile, according to one embodiment of the present disclosure, the processor 110 may acquire second bio-information for the user 1 while continuously acquiring first bio-information for the user 1. Furthermore, the processor 110 may update the neural network model by performing second learning on the neural network model 10 based on the acquired second bio-information and the first bio-information corresponding to the acquired second bio-information out of the continuously acquired first bio-information.

While continuously acquiring first bio-information, the processor 110 may acquire second bio-information for the user 1. The acquired second bio-information may be information measured from the user 1. This is distinct from second bio-information predicted by inputting the first bio-information to the neural network model 1. While continuously acquiring first bio-information, the processor 110 may receive second bio-information from an external computing device via the network unit 130. Alternatively, the processor 110 may receive second bio-information via a user interface.

In this case, the second bio-information may include the bio-information acquired from the user 1 based on an invasive measurement method or the bio-information acquired through direct measurement by a medical professional. Since this has been described in FIG. 1, a detailed description thereof will be omitted.

Meanwhile, when the second bio-information is acquired, the processor 110 may update the neural network model 10 by performing second learning on the neural network model 10 based on the acquired second bio-information. More specifically, the processor 110 may calibrate the pre-trained neural network model 10 to suit the user 1 based on the second bio-information and first bio-information corresponding to the second bio-information. In this case, the first bio-information corresponding to the second bio-information may be acquired within a preset time range from the time when the second bio-information was acquired. When the second bio-information is acquired, the processor 110 may identify the first bio-information acquired via the sensing unit 140 at the time when the second bio-information was acquired (more specifically, the time when the second bio-information was acquired by an external computing device) (or acquired within a preset time range from the time when the second bio-information was acquired), and may perform second learning on the neural network model 10 by using the first and second bio-information.

According to one embodiment of the present disclosure, the second learning may be supervised learning based on acquired second bio-information and first bio-information acquired corresponding to the acquired second bio-information. More specifically, the processor 110 may set the first bio-information corresponding to the second bio-information as input data and the second bio-information as a label, and may train the neural network model 10 on the relationship between the first and second bio-information. For example, the processor 110 may acquire predicted second bio-information based on the latent feature extracted for the first bio-information by inputting the first bio-information to the pre-trained neural network model 10, and may train the neural network model 10 to minimize the loss between the measured and acquired second bio-information and the predicted second bio-information based on a loss function. In this case, a contrastive loss function or an info NCE loss function may be utilized as the loss function. The processor 110 may adjust parameters of the pre-trained neural network model 10 via a backpropagation algorithm.

Meanwhile, referring to FIG. 4, for example, the first learning may be performed using acquired first bio-information each time the first bio-information is acquired, and the second learning may be performed using second bio-information and first bio-information corresponding to the second bio-information each time the second bio-information is acquired. In this case, when the second bio-information is acquired, the processor 110 may perform only the second learning for the neural network model 10 without performing the first learning. The reason for this is to increase the accuracy of the neural network model 10 by using the same first bio-information only for the second learning, which enables more accurate calibration of the neural network model 10, rather than using it for separate types of learning (the first and second learning). However, the learning is not limited thereto.

Meanwhile, referring to FIG. 5, the processor 110 may perform the first and second learning processes in parallel as the first and second bio-information are acquired. Meanwhile, referring to FIG. 5, the processor 110 may perform the first and second learning processes in parallel in steps S522 and S524 as the first and second bio-information are acquired in steps S521 and S523, respectively.

The processor 110 may perform first learning on the neural network model by using the periodically acquired first bio-information of the user 1 and perform second learning on the neural network model by using the acquired second bio-information based on Equations 1 to 3 below. More specifically, the processor 110 may update the neural network model based on gradient descent according to Equations 1 to 3 below. In this case, the processor 110 may train the neural network model by using the data of time step t with a batch size of 1.

$$\theta_{t+1} = \theta_t + \frac{\partial L}{\partial \theta}, \quad L = L_{cls} + L_{ssl} \tag{1}$$

$$L_{cls} = \begin{cases} 0, & if\ y\ is\ not\ given \\ CE\big(y_t, f(x_t, \theta)\big), & otherwise \end{cases} \tag{2}$$

$$L_{ssl} = \big\| g\big(f(x_t)\big) - x_t \big\|^2 \tag{3}$$

In this case, $\theta_t$ may be a parameter of the neural network model at time t. $x_t$ may be the first bio-information acquired at time t, and $y_t$ may be the second bio-information acquired at time t. L may be the overall loss function of the neural network model, $L_{cls}$ may be a supervised learning loss function, and $L_{ssl}$ may be a self-supervised learning loss function. $L_{cls}$ is defined as the cross-entropy loss $CE(y_t, f(x_t, \theta))$ between the second bio-information predicted based on the first bio-information acquired at time t and the second bio-information actually acquired at time t, and $L_{ssl}$ may be defined as a loss function that minimizes the Euclidean distance between $g(f(x_t))$, i.e., the result of the embedding of $f(x_t)$, and the input data $x_t$.

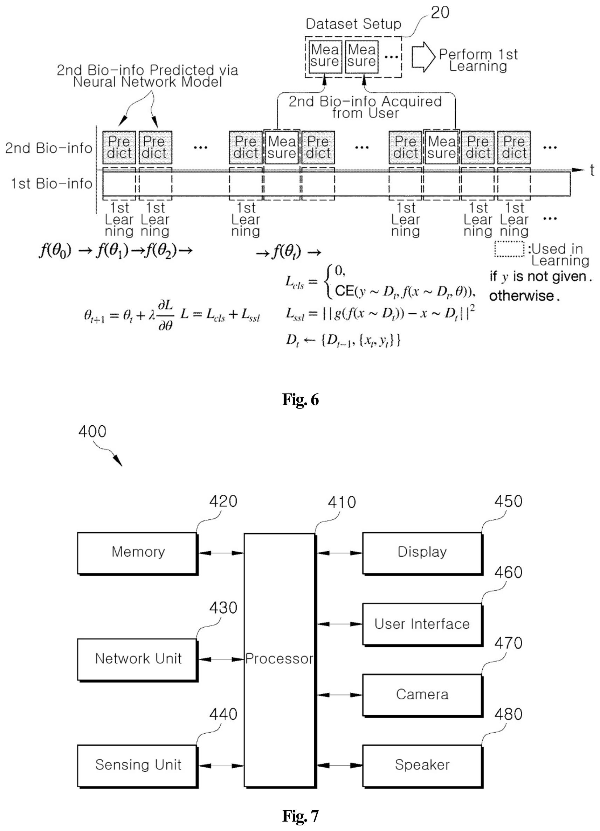

FIG. 6 is an exemplary diagram showing updating a neural network model based on first bio-information and a plurality of pieces of second bio-information included in a dataset 20 according to one embodiment of the present disclosure.

Meanwhile, according to one embodiment of the present disclosure, when second bio-information is acquired, the processor 110 may add the acquired second bio-information and first bio-information corresponding to the acquired second bio-information to the dataset 20 accumulated and acquired at a previous time, and may perform second learning on the neural network model based on the dataset 20, thereby updating the neural network model. In this case, the dataset 20 may include at least one second bio-information accumulated and acquired at a previous time and first bio-information corresponding to the at least one second bio-information, which are matched with each other.

More specifically, referring to FIG. 6, when new second bio-information is acquired, the processor 110 may identify the first bio-information acquired at the time when the second bio-information is acquired and add the second bio-information and the first bio-information to a dataset 20 accumulated and acquired at a previous time. In this case, the dataset 20 may include a plurality of pieces of second bio-information accumulated and acquired before the time when the new second bio-information is acquired, and a plurality of pieces of first bio-information corresponding to the plurality of pieces of second bio-information. That is, when the second bio-information is acquired, the processor 110 may update the dataset 20 with the acquired second bio-information and the first bio-information corresponding to the acquired second bio-information. Furthermore, the processor 110 may perform second learning on the neural network model 10 based on the dataset 20, thereby updating the neural network model 10.

In this case, the processor 110 may perform first learning on the neural network model by using the periodically acquired first bio-information of the user 1 and perform second learning on the neural network model by using the dataset 20 including the acquired plurality of pieces of second bio-information based on Equations 4 to 7 below. More specifically, the processor 110 may update the neural network model based on gradient descent according to Equations 4 to 7 below:

$$\theta_{t+1} = \theta_t + \frac{\partial L}{\partial \theta}, \quad L = L_{cls} + L_{ssl} \tag{4}$$

$$L_{cls} = \begin{cases} 0, & \text{if } y \text{ is not given} \\ CE\big(y_t{\sim}D_t, f(x_t{\sim}D_T, \theta)\big), & \text{otherwise} \end{cases} \tag{5}$$

$$L_{ssl} = \big\| g\big(f(x_t{\sim}D_t)\big) - x_t{\sim}D_t \big\|^2 \tag{6}$$

$$D_t \leftarrow \big\{ D_{t-1}, \{x_t, y_t\} \big\} \tag{7}$$

In this case, $\theta_t$ may be a parameter of the neural network model at time t. $x_t$ may be the first bio-information acquired at time t, and $y_t$ may be the second bio-information acquired at time t. L may be the overall loss function of the neural network model, $L_{cls}$ may be a supervised learning loss function, and $L_{ssl}$ may be a self-supervised learning loss function. $L_{cls}$ is defined as the cross-entropy loss $CE(y_t, f(x_t, \theta))$ between the second bio-information predicted based on the first bio-information acquired at time t and the second bio-information actually acquired at time t, and $L_{ssl}$ may be defined as a loss function that minimizes the Euclidean distance between $g(f(x_t))$, i.e., the result of the embedding of $f(xt)$, and the input data $x_t$. $D_t$ may be the dataset 20.

Meanwhile, the processor 110 may identify the bio-information of the user 1, and may share the dataset 20 related to second bio-information with another computing device 100 of another user 1 having bio-information similar to the identified bio-information. In this case, the bio-information may include the height, weight, age, gender, and/or the like of the user 1. For example, when the computing device 100 is a smartwatch, the processor 110 may acquire the bio-information of another user 1 from another smartwatch information of the other user 1. The processor 110 may identify the similarity between the bio-information of a plurality of other users 1 and the bio-information of the user 1, and may identify another computing device 100 of another user 1 having bio-information similar to the bio-information of the user 1 based on the identified similarity. Furthermore, the processor 110 may share the dataset 20 related to the second bio-information by transferring the acquired dataset 20 to the identified computing device 100. Alternatively, the processor 110 may also have the dataset 20 related to the second bio-information of another user 1 shared from another computing device 100 of the other user 1 having bio-information similar to that of the user 1. In particular, the second bio-information is acquired intermittently because it is measured non-invasively or by a medical professional, and the amount of data may be small. Accordingly, in the case of a plurality of users 1 having similar bio-information, the neural network model 10 may be updated to more rapidly reflect the characteristics of the user 1 therein by sharing the dataset 20 related to acquired second bio-information.

As an example, a server device operating in conjunction with the computing device 100 may acquire bio-information of the user 1 from the computing device 100, and may transmit the neural network model 10 corresponding to the acquired bio-information to the computing device 100. More specifically, the server device may store a plurality of neural network models 10 corresponding to a plurality of users 1. In particular, each of the neural network models 10 may correspond to a user (1) group including a plurality of users 1 having similar bio-information. Accordingly, when bio-information of the user 1 is received from the computing device 100, the server device may select bio-information similar to the received bio-information and transmit the neural network model 10 corresponding to the selected bio-information to the computing device 100. In this case, the server device may also include the user 1 of the computing device 100 in the user (1) group corresponding to the selected bio-information. The server device may share second bio-information acquired from each computing device 100 (or the dataset 20 set based on the second bio-information) for a plurality of users 1 included in the same user (1) group.

According to one embodiment of the present disclosure, the processor 110 may identify bio-information of the user 1, may cluster the user 1 and a plurality of other users 1 based on the identified bio-information, and may assign the dataset 20 including new data to each clustering group. More specifically, the processor 110 may acquire bio-information of a plurality of users 1 from a plurality of user terminal devices that operates in conjunction with the computing device 100. In this case, the processor 110 may cluster a plurality of users 1 based on bio-information of the plurality of users 1. Furthermore, the processor 110 may set the dataset 20 for each clustering group by using the second biosignals acquired from the user terminal devices included in each clustering group. That is, when second biosignals are acquired from each of multiple user (1) terminal devices included in a specific clustering group, the processor may collect the plurality of acquired biosignals, may set up the dataset 20 for the specific clustering group, and may assign the dataset 20 to the specific clustering group. In this case, the computing device 100 may be implemented as a server device that manages the health status of the plurality of users 1.

According to one embodiment of the present disclosure, the processor 110 may acquire second bio-information for the user 1 by inputting the periodically acquired first bio-information of the user 1 to the neural network model 10. This may be performed through the first learning process described above. Furthermore, the processor 110 may periodically monitor the trend of acquired second bio-information, and may update the neural network model 10 by performing second learning on the neural network model 10 based on the acquired second bio-information when a change in the trend of predicted second bio-information is detected. That is, when a change in the trend or tendency of second bio-information is detected, the processor 110 may determine to update the neural network model 10 based on the second bio-information. For example, when it is determined that the similarity between the trend of second bio-information in a previous cycle and the trend of second bio-information in a current cycle is less than a preset value, the processor 110 may identify that the trend of second bio-information has changed and determine to perform second learning on the neural network model 10 by using the second bio-information.

According to one embodiment of the present disclosure, when first bio-information is acquired, the processor 110 may predict second bio-information by inputting the acquired first bio-information to the pre-trained neural network model 10, and may output the predicted second bio-information. For example, the processor 110 may output the predicted second bio-information via the display or output interface of the computing device 100. This process may be performed in parallel with the first learning process, or may be performed after the first learning process based on the acquired first bio-information has been completed.

According to one embodiment of the present disclosure, when second bio-information is acquired, the processor 110 may acquire predicted second bio-information as output data by inputting first bio-information corresponding to the second bio-information to the neural network model 10. Furthermore, the processor 110 may identify the error by comparing the predicted second bio-information and the acquired second bio-information. When the error between the second bio-information acquired as the output data and the second bio-information acquired from another computing device 100 is smaller than a preset value, the processor 110 may determine to stop the second learning. The reason for this is to determine that the process of personalizing the neural network model 10 for the user 1 has been completed, thereby reducing the resources of the computing device 100 required for learning.

FIG. 7 is a block diagram of a computing device 400 according to another embodiment of the present disclosure.

Referring to FIG. 7, the computing device 400 according to the other embodiment of the present disclosure includes a processor 410, memory 420, a network unit 430, a sensing unit 440, a display 450, a user interface 460, a camera 470, and a speaker 480. Among the components shown in FIG. 7, the processor 410, the memory 420, the network unit 430, and the sensing unit 440 correspond to the processor 110, memory 120, network unit 130, and sensing unit 140 of the computing device 100 shown in FIG. 2, so that detailed descriptions thereof will be omitted.

The display 450 may display various images. The images include both still images and moving images. The display 450 may output activity-related guidance information generated based on the status of the user 1. The display 450 may be implemented in the form of various types of displays, such as an liquid crystal display (LCD) panel, an organic light emitting diode (OLED) display, a liquid crystal on silicon (LCoS) display, a digital light processing (DLP) display, and the like. Furthermore, the display 450 may also include a driving circuit, a backlight unit, and the like, which can be implemented in the form of elements such as a-Si TFTs, low temperature poly silicon (LTPS) TFTs, and organic TFTs (OTFTs).

Meanwhile, the display 450 may be implemented as a touch screen in combination with a touch panel. In this case, the display 450 may function not only as an output interface configured to output images via the touch screen, but also as an input interface configured to receive touch input from the user 1.

The user interface 460 is a component used by the computing device 100 to interact with the user 1. The user interface 460 may include, but is not limited to, at least one of a touch sensor, a motion sensor, buttons, a jog dial, and switches. The processor 410 may receive second bio-information and the bio-information (e.g., the occupation, age, and gender) of the user 1 via the user interface 460.

The camera 470 acquires an image of an object near the user 1 by capturing the image of the object. More specifically, the camera 470 may capture an image of food consumed by the user 1. In this case, the processor 410 may determine the nutritional status of the user 1 and provide recommended meal information as guidance information based on the status of the user 1 and the image of the food consumed. For this purpose, the camera 470 may be implemented as an imaging device such as a CMOS image sensor (CIS) having a CMOS structure or a charge-coupled device (CCD) having a CCD structure. However, the camera 470 is not limited thereto, and may be implemented as camera modules having various resolutions capable of capturing subjects. Meanwhile, the camera 470 may be implemented as a depth camera (e.g., an IR depth camera), a stereo camera, or an RGB camera.

The speaker 480 is a component that outputs various audio data that has undergone various processing operations, such as decoding, amplification, and noise filtering, via an audio processing unit (not shown). The speaker 480 may output various types of notification sounds or voice messages. According to one embodiment of the present disclosure, the processor 410 may convert an electrical signal received from an external device into the voice of the user 1 and output it via the speaker 480. For example, the speaker 480 may output a voice message warning of a disease or suggesting a diagnosis of a disease related with the second bio-information identified via the neural network model 10.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as single may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

**Claims**

1. A method of acquiring a user-customized neural network model that predicts bio-information of a user, the method being performed by a computing device including at least one processor, the method comprising:

   acquiring a neural network model trained to predict second bio-information of a different type from that of first bio-information based on the first bio-information; and
   based on new data for the user being acquired, updating the neural network model based on the acquired new data so that the neural network model is personalized for the user;
   wherein the new data comprises at least one of first bio-information for the user and second bio-information for the user.

2. The method of claim 1, wherein the updating comprises:

   continuously acquiring first bio-information for the user; and
   updating the neural network model by performing first learning on the neural network model based on the acquired first bio-information.

3. The method of claim 1, wherein the updating comprises:

   acquiring second bio-information for the user while continuously acquiring first bio-information for the user; and
   updating the neural network model by performing second learning on the neural network model based on the acquired second bio-information and first bio-information corresponding to the acquired second bio-information out of the continuously acquired first bio-information.

4. The method of claim 3, wherein updating the neural network model by performing second learning on the neural network model comprises performing the second learning on the neural network model based on the acquired second bio-information and first bio-information corresponding to the acquired second bio-information each time the second bio-information is acquired;

5. The method of claim 3, wherein updating the neural network model by performing second learning on the neural network model comprises:

   when the second bio-information is acquired, updating a dataset accumulated and acquired at a previous time by adding the acquired second bio-information and first bio-information corresponding to the acquired second bio-information to the dataset; and
   performing second learning on the neural network model based on the updated dataset;
   wherein the dataset includes at least one piece of second bio-information accumulated and acquired at the previous time and at least one first piece bio-information corresponding to the at least one piece of second bio-information, which are matched with each other.

6. The method of claim 3, wherein the first bio-information corresponding to the second bio-information is acquired within a preset time range from a time when the second bio-information was acquired.

7. The method of claim 3, comprising acquiring predicted second bio-information by inputting first bio-information corresponding to the acquired second bio-information to the neural network model, and determining whether to stop a process of updating the neural network model by comparing the acquired second bio-information with the predicted second bio-information.

8. The method of claim 1, comprising identifying biological information of the user, clustering the user and a plurality of other users based on the identified biological information, and assigning a dataset, including the new data, to each cluster.

9. The method of claim 2, wherein the first learning is a self-supervised learning method based on the acquired first bio-information.

10. The method of claim 3, wherein the second learning is a supervised learning method based on the acquired second bio-information and first bio-information corresponding to the acquired second bio-information.

11. The method of claim 1, wherein:

the first bio-information comprises bio-information acquired from the user based on a non-invasive measurement method; and
the second bio-information comprises bio-information acquired from the user based on an invasive measurement method or bio-information acquired through direct measurement by a medical professional.

12. A method of diagnosing a disease of a user based on a user-customized neural network model, the method being performed by a computing device including at least one processor, the method comprising:

acquiring first bio-information; and
identifying second bio-information of a different type from that of the first bio-information by inputting the acquired first bio-information to a pre-trained neural network model;
wherein the pre-trained neural network model is updated based on new data each time the new data for the user is acquired; and
wherein the new data comprises at least one of first bio-information for the user and second bio-information for the user.

13. A computer program stored in a computer-readable storage medium, the computer program causing operations for acquiring a user-customized neural network model that predicts bio-infonnation of a user to be performed when executed by at least one processor, wherein the operations comprise operations of:

acquiring a neural network model trained to predict second bio-information of a different type from that of first bio-information based on the first bio-information; and
based on new data for the user being acquired, updating the neural network model based on the acquired new data so that the neural network model is personalized for the user;
wherein the new data comprises at least one of first bio-information for the user and second bio-information for the user.

14. A computing device for acquiring a user-customized neural network model that predicts bio-information of a user, the computing device comprising:

memory including program codes;
a network unit;
a sensing unit configured to detect first bio-information of a user; and
a processor configured to acquire a neural network model trained to predict second bio-information of a different type from that of first bio-information based on the first bio-information, and to, based on new data for the user being acquired, update the neural network model based on the acquired new data so that the neural network model is personalized for the user;
wherein the new data comprises at least one of first bio-information for the user and second bio-information for the user.

① Acquire Neural Network Model

④ Update Neural Network Model Based on At Least One of 1st Bio-Info and 2nd Bio-Info

② Acquire 1st Bio-Info for User

③ Acquire 2nd Bio-Info for User

t1 ... t2 TIME

**Fig. 1**

Computing Device

At Least One Processor — 110

Memory — 120

Network Unit — 130

Sensing Unit — 140

100

**Fig. 2**

**Fig. 3**

$$\theta_{t+1} = \theta_t + \lambda \frac{\partial L}{\partial \theta} \quad L = L_{cls} + L_{ssl}$$

$$L_{cls} = \begin{cases} 0, & \text{if } y \text{ is not given}. \\ \text{CE}(y_t, f(x_t, \theta)), & \text{otherwise}. \end{cases}$$

$$L_{ssl} = ||g(f(x_t)) - x_t||^2$$

**Fig. 4**

START

Acquire Neural Network Model
Trained to Predict 2nd Bio-info
of Different Type from That of
1nd Bio-info Based on 1st
Bio-info — S510

Acquire 1st Bio-info for User
via Sensing Unit — S521

Acquire 2nd Bio-info for User
via Network Unit — S523

Perform 1st Learning on Neural
Network Model Based on
Acquired 1st Bio-info — S522

Perform 2nd Learning on
Neural Network Model Based
on Acquired 2nd Bio-info and
Corresponding 1st Bio-info — S524

END

**Fig. 5**

$$f(\theta_0) \rightarrow f(\theta_1) \rightarrow f(\theta_2) \rightarrow \qquad \rightarrow f(\theta_t) \rightarrow$$

$$L_{cls} = \begin{cases} 0, & \text{if } y \text{ is not given.} \\ CE(y \sim D_t, f(x \sim D_t, \theta)), & \text{otherwise.} \end{cases}$$

$$\theta_{t+1} = \theta_t + \lambda \frac{\partial L}{\partial \theta} \quad L = L_{cls} + L_{ssl} \qquad L_{ssl} = ||g(f(x \sim D_t)) - x \sim D_t||^2$$

$$D_t \leftarrow \{D_{t-1}, \{x_t, y_t\}\}$$

**Fig. 6**

**Fig. 7**

## INTERNATIONAL SEARCH REPORT

| International application No. |
| --- |
| **PCT/KR2024/007392** |

| **A.** | **CLASSIFICATION OF SUBJECT MATTER** |
| --- | --- |

**G16H 10/60**(2018.01)i; **G16H 50/20**(2018.01)i; **G16H 40/60**(2018.01)i; **A61B 5/021**(2006.01)i; **A61B 5/00**(2006.01)i; **G06N 3/0464**(2023.01)i; **G06N 3/044**(2023.01)i; **G06N 3/0475**(2023.01)i

According to International Patent Classification (IPC) or to both national classification and IPC

| **B.** | **FIELDS SEARCHED** |
| --- | --- |

Minimum documentation searched (classification system followed by classification symbols)

G16H 10/60(2018.01); A61B 5/00(2006.01); A61B 5/145(2006.01); G06N 20/00(2019.01); G06Q 50/22(2012.01); G16H 50/20(2018.01); G16H 50/30(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 생체정보(biometrics), 신경망(neural network), 업데이트(update), 학습(learning), 모니터링(monitoring), 의료(medical)

| **C.** | **DOCUMENTS CONSIDERED TO BE RELEVANT** | |
| --- | --- | --- |

| Category* | Citation of document, with indication, where appropriate, of the relevant passages | Relevant to claim No. |
| --- | --- | --- |
| Y | KR 10-2043376 B1 (KOREA INSTITUTE OF SCIENCE AND TECHNOLOGY) 11 November 2019 (2019-11-11)<br>See claims 1-3. | 1-14 |
| Y | KR 10-2020-0127470 A (SAMSUNG ELECTRONICS CO., LTD.) 11 November 2020 (2020-11-11)<br>See paragraph [0047] and claims 1, 8-10 and 13. | 1-14 |
| Y | KR 10-2464709 B1 (POSTECH RESEARCH AND BUSINESS DEVELOPMENT FOUNDATION) 09 November 2022 (2022-11-09)<br>See paragraph [0016] and claims 1 and 8. | 3-7,10 |
| Y | US 2022-0044817 A1 (RIKEN) 10 February 2022 (2022-02-10)<br>See claim 41. | 8 |
| Y | KR 10-2414769 B1 (DABLE HEALTHCARE. CORP.) 30 June 2022 (2022-06-30)<br>See claim 6. | 9-10 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **20 September 2024** | **20 September 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/007392**

| Patent document cited in search report | | | Publication date (day/month/year) | Patent family member(s) | | | Publication date (day/month/year) |
|---|---|---|---|---|---|---|---|
| KR | 10-2043376 | B1 | 11 November 2019 | US | 11317840 | B2 | 03 May 2022 |
| | | | | US | 2020-0054262 | A1 | 20 February 2020 |
| KR | 10-2020-0127470 | A | 11 November 2020 | EP | 3734613 | A1 | 04 November 2020 |
| | | | | US | 11399746 | B2 | 02 August 2022 |
| | | | | US | 2020-0345281 | A1 | 05 November 2020 |
| KR | 10-2464709 | B1 | 09 November 2022 | KR | 10-2020-0119216 | A | 19 October 2020 |
| US | 2022-0044817 | A1 | 10 February 2022 | CA | 3118435 | A1 | 07 May 2020 |
| | | | | CN | 113168918 | A | 23 July 2021 |
| | | | | EP | 3876184 | A1 | 08 September 2021 |
| | | | | JP | 2021-103582 | A | 15 July 2021 |
| | | | | JP | 2024-074846 | A | 31 May 2024 |
| | | | | JP | 6864947 | B2 | 28 April 2021 |
| | | | | JP | 7466914 | B2 | 15 April 2024 |
| | | | | WO | 2020-091053 | A1 | 07 May 2020 |
| KR | 10-2414769 | B1 | 30 June 2022 | None | | | |

Form PCT/ISA/210 (patent family annex) (July 2022)